# EUROPEAN PATENT SPECIFICATION

(11) **EP 2 084 292 B1**
(45) Date of publication and mention of the grant of the patent: **23.02.2011**
(21) Application number: 07824028.0
(22) Date of filing: 05.10.2007
(51) Int. Cl.: C12Q 1/00

(54) **A REAGENT FORMULATION USING RUTHENIUM HEXAMINE AS A MEDIATOR FOR ELECTROCHEMICAL TEST STRIPS**
REAGENSFORMULIERUNG MIT RUTHENIUMHEXAMIN ALS VERMITTLER FÜR ELEKTROCHEMISCHE TESTSTREIFEN
FORMULATION DE RÉACTIF UTILISANT DE L'HEXAMINE DE RUTHÉNIUM EN TANT QUE MÉDIATEUR POUR DES BANDES DE TESTS ÉLECTROCHIMIQUES

(30) Priority: 05.10.2006 US 850221 P
(43) Date of publication of application: 05.08.2009
(73) Proprietor: Lifescan Scotland Limited, Inverness-Shire IV2 3ED (GB)
(72) Inventor: CARDOSI, Marco, F., Croy, Inverness IV2 5FP (GB); MILLS, Leanne, Croy, Inverness-shire IV2 5PA (GB); LEACH, Christopher, Philip, Inverness, Invernessshire IV2 3DP (GB)
(74) Representative: Tunstall, Christopher Stephen
(86) International application number: PCT/GB2007/003772
(87) International publication number: WO 2008/040984

(56) References cited:
- EP-A- 1 426 757
- EP-A- 1 522 592
- WO-A-2006/057722
- CHEN L ET AL: "Bioinorganic composites for enzyme electrodes." ANAL CHEM, vol. 73, no. 13, 1 July 2001 (2001-07-01), pages 2862-2868, XP002463785
- GANG C ET AL: "Disposable amperometric glucose sensor electrode with enzyme-immobilized nitrocellulose strip" TALANTA, vol. 54, 2001, pages 1105-1111, XP002972399
- MORRIS N A ET AL: "AN ELECTROCHEMICAL CAPILLARY FILL DEVICE FOR THE ANALYSIS OF GLUCOSE INCORPORATING GLUCOSE OXIDASE AND RUTHENIUM (III) HEXAMINE AS MEDIATOR" ELECTROANALYSIS, vol. 4, no. 1, 1992, pages 1-9, XP002065769

## Description

### 1. Priority

This application claims the benefits of priority under 35 U.S.C. § 119 from provisional application S.N. 60/850,221 filed on October 5, 2006, entitled: "A REAGENT FORMULATION USING A RUTHENIUM BASED MEDIATOR FOR ELECTROCHEMICAL TEST STRIPS, "

### 2. Description of the Related Art

EP 1426757 discusses a concentration test instrument including a substrate, first and second electrodes formed on the substrate, and a reagent layer formed as a solid. The reagent layer contains an oxidation-reduction enzyme and a Ru compound.

WO 2006/057722 discusses biosensors for measuring analyte concentration in a bodily fluid comprising at least one electrode comprising semiconducting, conducting, or thin film carbon material, and an electron mediator comprising a ruthenium containing electron mediator, or a ferricyanide material or ferrocene carboxylic acid

EP 1522592 discusses a glucose sensor in which glucose dehydrogenase with cytochrome C attached thereto or glucose dehydrogenase derived from derived from a microorganism belonging to a burkholderia genus is used as the enzyme, and a Ru compound is used as the electron carrier.

CHEN L. ET AL: "Bioinorganic composites for enzyme electrodes." ANAL CHEM, vol. 73, no. 13, 1 July 2001 (2001-07-01), pages 2862-2868, XP002463785, discusses combining sparingly soluble redox salts with glucose oxidase in a host matrix of a biopolymer chitosan to form bioinorganic composite films on the surface of glassy carbon electrodes.

GANG C ET AL: "Disposable amperometric glucose sensor electrode with enzyme-immobilized nitrocellulose strip" TALANTA, vol. 54, 2001, pages 1105-1111, XP002972399, discusses the examination of electromechanical properties of screenprinted carbon past electrodes with a glucose oxidase-immobilized and hexamine ruthenium (III) chloride containing nitrocellulose strip.

Electrochemical glucose test strips, such as those used in the OneTouch® Ultra® whole blood testing kit, which is available from LifeScan, Inc., are designed to measure the concentration of glucose in a blood sample from patients with diabetes. The measurement of glucose is based upon the specific oxidation of glucose by the enzyme glucose oxidase (GO). The reactions which may occur in a glucose test strip are summarized below in Equations 1 and 2.

Glucose + GO₍ₒₓ₎ **→** Gluconic Acid + GO_{(red)} Eq. 1

GO_{(red)} + 2 Fe(CN)₆³⁻ → GO₍ₒₓ₎ + 2 Fe(CN)₆⁴⁻ Eq. 2

As illustrated in Equation 1, glucose is oxidized to gluconic acid by the oxidized form of glucose oxidase (GO₍ₒₓ₎). It should be noted that GO₍ₒₓ₎ may also be referred to as an "oxidized enzyme." During the reaction in Equation 1, the oxidized enzyme GO₍ₒₓ₎ is converted to its reduced state which is denoted as GO_{(red)} (i.e., "reduced enzyme"). Next, the reduced enzyme GO_{(red)} is re-oxidized back to GO₍ₒₓ₎ by reaction with Fe(CN)₆³⁻ (referred to as either the oxidized mediator or ferricyanide) as illustrated in Equation 2. During the re-generation of GO_{(red)} back to its oxidized state GO₍ₒₓ₎, Fe(CN)₆³⁻ is reduced to Fe(CN)₆⁴⁻ (referred to as either reduced mediator or ferrocyanide).

When the reactions set forth above are conducted with a test voltage applied between two electrodes, a test current may be created by the electrochemical re-oxidation of the reduced mediator at the electrode surface. Thus, since, in an ideal environment, the amount of ferrocyanide created during the chemical reaction described above is directly proportional to the amount of glucose in the sample positioned between the electrodes, the test current generated would be proportional to the glucose content of the sample. A mediator, such as ferricyanide, is a compound that accepts electrons from an enzyme such as glucose oxidase and then donates the electrons to an electrode. As the concentration of glucose in the sample increases, the amount of reduced mediator formed also increases; hence, there is a direct relationship between the test current, resulting from the re-oxidation of reduced mediator, and glucose concentration. In particular, the transfer of electrons across the electrical interface results in the flow of a test current (2 moles of electrons for every mole of glucose that is oxidized). The test current resulting from the introduction of glucose may, therefore, be referred to as a glucose current.

Because it can be very important to know the concentration of glucose in blood, particularly in people with diabetes, test meters have been developed using the principals set forth above to enable the average person to sample and test their blood for determining their glucose concentration at any given time. The glucose current generated is detected by the test meter and converted into a glucose concentration reading using an algorithm that relates the test current to a glucose concentration via a simple mathematical formula. In general, the test meters work in conjunction with a disposable test strip that includes a sample receiving chamber and at least two electrodes disposed within the sample receiving chamber in addition to the enzyme (e.g. glucose oxidase) and the mediator (e.g. ferricyanide). In use, the user pricks their finger or other convenient site to induce bleeding and introduces a blood sample to the sample receiving chamber, thus starting the chemical reaction set forth above.

In electrochemical terms, the function of the meter is two fold. Firstly, it provides a polarizing voltage (approximately 400 mV in the case of OneTouch® Ultra®) that polarizes the electrical interface and allows current flow at the carbon working electrode surface. Secondly, it measures the current that flows in the external circuit between the anode (working electrode) and the cathode (reference electrode). The test meter may, therefore be considered to be a simple electrochemical system that operates in a two-electrode mode although, in practice, third and, even fourth electrodes may be used to facilitate the measurement of glucose and/or perform other functions in the test meter.

As previously described, the amount of reduced mediator should be proportional to the glucose concentration present in a physiological fluid based on the reactions described in Equation 1 & 2. Under certain circumstances, oxidized mediators such as, for example, ferricyanide can be converted to ferrocyanide (reduced mediator) in the presence of a humid environment. The generation of reduced mediator by non-glucose dependent reactions may cause a falsely elevated glucose concentration to be measured which, in turn, affects the assay's accuracy.

The presence of interfering compounds may also cause the test current to be falsely elevated because the interfering compound may become oxidized at the working electrode. Additionally, the oxidized mediator may become reduced by the interfering compound where the resulting reduced mediator can become oxidized at the working electrode. One strategy for decreasing the effects of interfering compounds is to use a relatively low test voltage between the working electrode and reference electrode. In order to employ a lower test voltage, the reagent formulation requires the mediator to have a lower redox voltage.

As such, applicants recognize that there is a great need for using mediators which do not convert to the reduced state in the presence of humidity and have a relatively low redox voltage. Further, such mediators need to be incorporated into a reagent formulation that can be easily coated onto a test strip in a robust manner enabling accurate and precise glucose measurements.

### SUMMARY OF INVENTION

In one embodiment, a test strip is provided which is capable of measuring an analyte. The test strip may include a working electrode and a reference electrode where the reagent formulation is disposed on the working electrode. The reagent formulation may be coated onto the test strip. The reagent formulation includes an enzyme, a ruthenium hexamine mediator, and a solution for dissolving the enzyme and the ruthenium hexamine mediator. The ruthenium hexamine has a concentration range from 15% to 20% (weight of mediator / volume) of solution. The enzyme is selected from the group consisting of glucose oxidase and glucose dehydrogenase. When using glucose oxidase, the enzyme activity per unit volume may range from about 1500 units/mL to about 8000units/mL. The solution for dissolving the enzyme may be a buffer such as, for example, phosphate, citrate, or citraconate. When using phosphate buffer, the pH may be about 7.

In another embodiment, the reagent formulation may further include a filler having hydrophilic and hydrophobic domains. In one embodiment, the filler may be a silica such as for example Cab-o-Sil TS 610. The formulation may be disposed on the working electrode using a process of screen printing. The screen may have a frame which secures a plurality of interwoven threads. The plurality of interwoven threads may form a plurality of open rectangular spaces for allowing the reagent formulation to pass therethrough. The plurality of interwoven threads may have a thread spacing and a thread diameter. The thread spacing may range from about 90 threads per centimeter to about 120 threads per centimeter. The thread diameter may range from about 30 microns to about 50 microns.

In another aspect, a test strip includes a substrate, two electrodes and a reagent. The planar substrate extends from a first end to a second end. The first and second electrodes are disposed on the substrate proximate one of the first and second ends with a reagent layer disposed thereon. The reagent layer has ruthenium hexamine trichloride in a buffer solution with the ruthenium hexamine trichloride comprising a concentration range from 15% to 20% (weight / volume).

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

Figure 1 illustrates a top exploded perspective view of a prior art embodiment of an unassembled test strip;

Figure 2 illustrates a top plan view of the prior art test strip as shown in Figure 1 after it has been assembled;

Figure 3 illustrates a top plan view of a test meter connected to the prior art test strip as shown in Figures 1 and 2;

Figure 4 illustrates a simplified schematic view of the test meter of Figure 3 forming an electrical connection with the test strip of Figures 1 and 2;

Figure 5a is a graph illustrating the application of an applied test voltage, from the test meter of Figure 3, to the test strip of Figures 1 and 2, for a test time interval t_{T} for generating a test current which can be used for calculating an analyte concentration;

Figure 5b is a graph illustrating an alternative embodiment for applying test voltages and an open-circuit time interval, from the test meter of Figure 3, to the test strip of Figures 1 and 2, for a test time interval t_{T} for generating a test current which can be used for calculating an analyte concentration;

Figure 5c is an expanded view of the graph of Figure 5b illustrating a serial application of an applied test voltage for detecting fluid, an open-circuit time interval, and another applied test voltage for measuring an analyte concentration;

Figure 6 is a graph illustrating a test current which results from the applied test voltage of Figure 5a when a blood sample is applied to the test strip of Figures 1 and 2;

Figure 7 illustrates a top exploded perspective view of an unassembled test strip in another embodiment;

Figure 8 is a graph showing the average bias to reference measurement of a test strip measurement for test strips using a ferricyanide based reagent layer stored for seven days at room temperature in a desiccated environment (filled in circles) or at 40 °C in a 75% relative humidity (RH) (open squares);

Figure 9 is a graph showing the average bias to reference measurement of a test strip measurement for test strips using a ruthenium based reagent layer stored for seven days at room temperature in a desiccated environment (filled in circles) or at 40 °C in a 75% relative humidity (RH) (open squares);

Figure 10 is a graph showing the average bias to reference measurement of a test strip measurement for test strips using a using either ferricyanide based reagent layer (open triangles) or a ruthenium based reagent layer (filled in diamonds) where the blood samples had varying concentrations of uric acid; and

Figure 11 is a graph showing the average bias to reference measurement of a test strip measurement for test strips using a using either ferricyanide based reagent layer (open triangles) or a ruthenium based reagent layer (filled in diamonds) where the blood samples had varying concentrations of gentisic acid.

### DETAILED DESCRIPTION OF ILLUSTRATIVE EMBODIMENTS OF THE

### INVENTION

Embodiments of the present invention are directed to a reagent formulation for use in electrochemical test strips. In particular, the embodiments include the use of ruthenium hexamine as a mediator to enable the manufacture of test strips which have increased stability under high humidity environmental conditions and/or also have decreased oxidation of interfering compounds. In an embodiment, a reagent formulation is described which may be easily coated onto a test strip in a robust manner enabling accurate and precise glucose measurements.

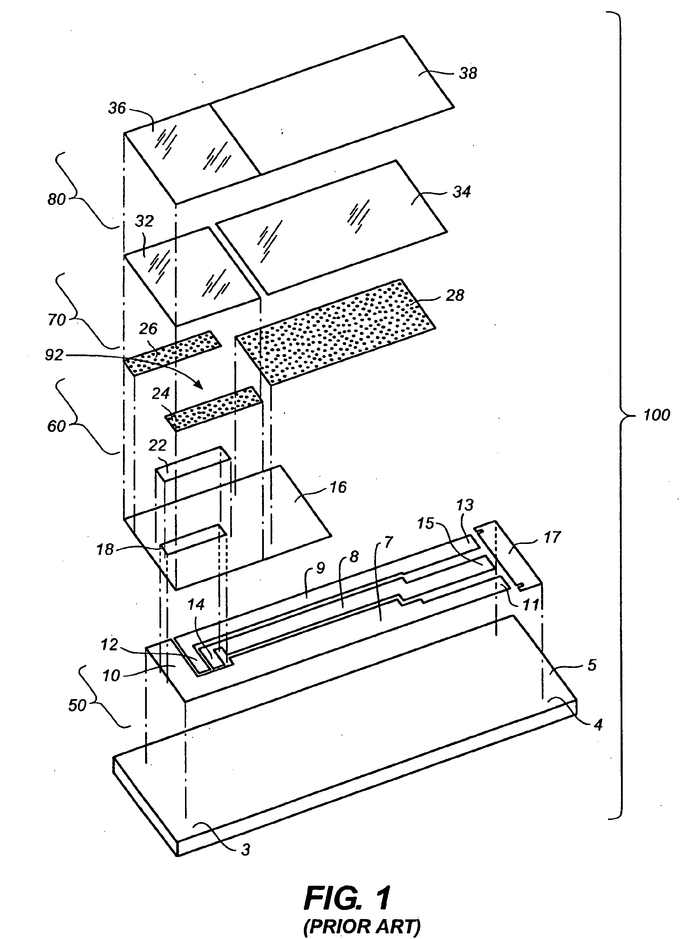

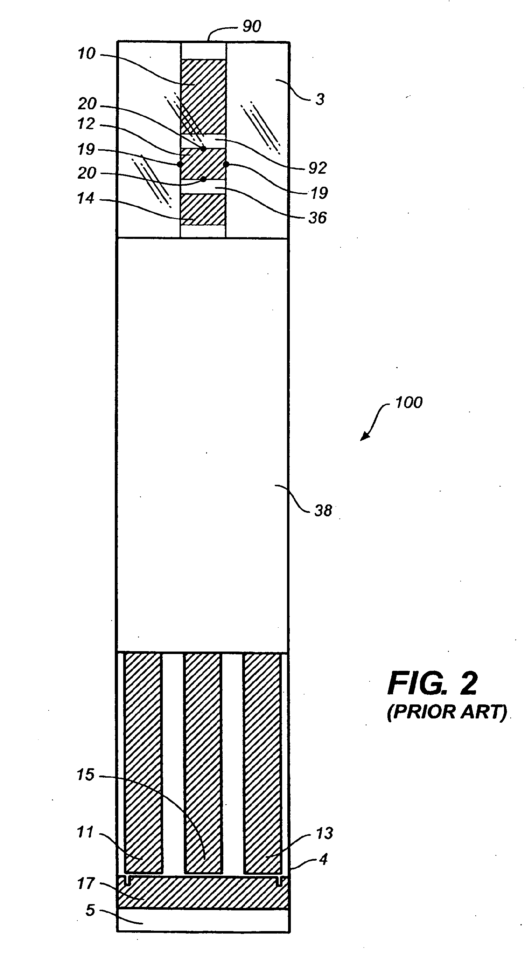

Figure 1 is an exploded perspective view of a prior art test strip 100, which includes six layers disposed on a substrate 5. These six layers may be a conductive layer 50, an insulation layer 16, a reagent layer 22, an adhesive layer 60, a hydrophilic layer 70, and a top layer 80. Test strip 100 may be manufactured in a series of steps wherein the conductive layer 50, insulation layer 16, reagent layer 22, adhesive layer 60 are sequentially deposited on substrate 5 using, for example, a screen printing process as described in U.S. Pre-Grant Publication No. US20050096409A1 and published International Application No.'s WO2004040948A1, WO2004040290A1, WO2004040287A1, WO2004040285A2, WO2004040005A1, WO2004039897A2, and WO2004039600A2. In an alternative embodiment, an ink jetting process may be used to deposit reagent layer 22 which is described in U.S. Patent No. 6,179,979. Hydrophilic layer 70 and top layer 80 may be disposed from a roll stock and laminated onto substrate 5. Test strip 100 has a distal portion 3 and a proximal portion 4 as shown in Figures 1 and 2.

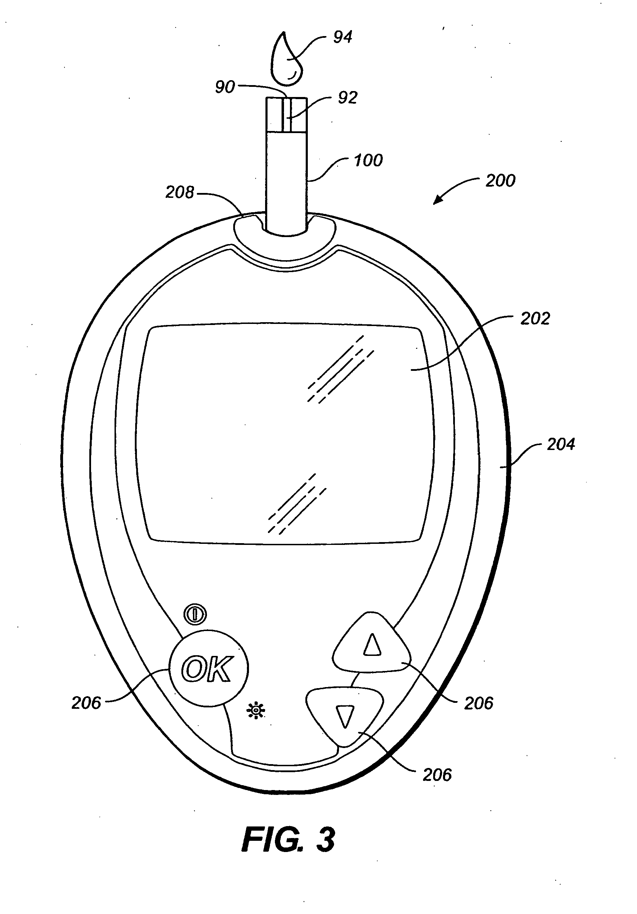

The fully assembled test strip 100, as shown in Figure 2, includes an inlet 90 through which a blood sample may be drawn into a sample receiving chamber 92. Inlet 90 may be formed by cutting through a distal portion 3 of test strip 100. A blood sample 94 can be applied to inlet 90, as illustrated in Figure 3, to fill a sample receiving chamber 92 so that glucose can be measured. The side edges of a first adhesive pad 24 and a second adhesive pad 26 located adjacent to reagent layer 22 each define a wall of sample receiving chamber 92. A bottom portion or "floor" of sample receiving chamber 92 includes a portion of substrate 5, conductive layer 50, and insulation layer 16. A top portion or "proof" of sample receiving chamber 92 includes distal hydrophilic portion 32.

For test strip 100, as illustrated in Figure 1, conductive layer 50 includes a reference electrode 10, a first working electrode 12, a second working electrode 14, a first contact 13, a second contact 15, a reference contact 11, a first working electrode track 8, a second working electrode track 9, a reference electrode track 7, and a strip detection bar 17. The conductive layer may be a carbon ink such as the one described in U.S. Patent No. 5,653,918. First contact 13, second contact 15, and reference contact 11 may be adapted to electrically connect to test meter 200. First working electrode track 8 provides an electrically continuous pathway from first working electrode 12 to first contact 13. Similarly, second working electrode track 9 provides an electrically continuous pathway from second working electrode 14 to second contact 15. Similarly, reference electrode track 7 provides an electrically continuous pathway from reference electrode 10 to reference contact 11.

In Figure 1, insulation layer 16 includes an aperture 18 which exposes a portion of reference electrode 10, first working electrode 12, and second working electrode 14 which can be wetted by a liquid sample. As an example, insulation layer 16 may be Ercon E6110-116 Jet Black Insulayer™ ink which may be purchased from Ercon, Inc (Waltham, Massachusetts).

Reagent layer 22 may be disposed on a portion of conductive layer 50, substrate 5, and insulation layer 16 as illustrated in Figure 1. Reagent layer 22 may include chemicals such as an enzyme and a mediator which selectivity reacts with glucose. An example of an enzyme may be glucose oxidase and an example of a mediator may be ferricyanide.

Enzymes for use in this invention include either glucose oxidase or glucose dehydrogenase. More specifically, the glucose dehydrogenase may have a pyrrylo-quinoline quinone co-factor (abbreviated as PQQ or may be referred to its common name which is methoxatin). Mediator for use in this invention is ruthenium hexamine trichloride ([Ru^{III}(NH₃)₆]Cl₃ and may also be simply referred to as ruthenium hexamine). During the reactions as illustrated in Equations 1 and 2, a proportional amount of reduced mediator can be generated that is electrochemically measured for calculating a glucose concentration. Examples of reagent formulations or inks suitable for use in the embodiments can be found in US patents 5,708,247 6,046,051, and 6,241,862; U.S. Pre-Grant Publication No. 20030217918A1; published international applications WO01/67099 and WO01/73124.

Reagent layer 22 may be formed from an enzyme ink or formulation, which is disposed onto a conductive layer and dried. An enzyme ink or formulation typically contains a liquid, such as a buffer, for dispersing and/or dissolving materials used for the electrochemical detection of an analyte such as glucose. Buffers which may be suitable for the formulation can be phosphate, citrate and citraconate.

In an embodiment, the formulation may include a 200 mM phosphate buffer having a pH of about 7 and a ruthenium hexamine mediator concentration ranging from 15% to 20% (percentage based on weight of mediator / volume of buffer). The pH of around 7 was chosen because glucose oxidase has a sufficiently high activity at this pH when using ruthenium hexamine as a mediator. When the enzyme ink is formulated to have greater than a 20% ruthenium hexamine concentration, solid particles of ruthenium hexamine may be present in reagent layer 22 which do not dissolve during testing. The presence of undissolved ruthenium hexamine may cause a decrease in the test strip-to-test strip precision. When the enzyme ink is formulated to have less than a 15% ruthenium hexamine concentration, the magnitude of the test current values may decrease with the concentration of ruthenium hexamine. In general, it is undesirable for the magnitude of the test current values to be dependent on the concentration of ruthenium hexamine because small changes in ruthenium hexamine concentration will cause variability in the test current values and, in turn, the strip lot-to-strip lot variability.

In an embodiment, the formulation may have an enzyme activity ranging from about 1500 units/mL to about 8000 units/mL. The enzyme activity range may be selected so that the glucose current does not depend on the level of enzyme activity in the formulation so long as the enzyme activity level is within the above stated range. The enzyme activity should be sufficiently large to ensure that the resulting glucose current will not be dependent on small variations in the enzyme activity. For instance, the glucose current will depend on the amount of enzyme activity in the formulation if the enzyme activity is less than 1500 units/mL. On the other hand, for enzyme activity levels greater than 8000 units/mL, solubility issues may arise where the glucose oxidase cannot be sufficiently dissolved in the formulation. Glucose oxidase may be commercially available from Biozyme Laboratories International Limited (San Diego, California, U.S.A.). The glucose oxidase may have an enzyme activity of about 250 units/mg using where the enzyme activity units are based on an o-dianisidine assay at pH 7 and 25 °C.

An enzyme ink which contains a filler having both hydrophobic and hydrophilic domains may be disposed onto the working electrode using a screen printing process. An example of a filler may be a silica such as, for example, Cab-o-Sil TS 610 which is commercially available from Cabot Inc., Boston, Massachusetts. Typically, a screen may be in the form of a rectangular frame which secures a plurality of interwoven threads. The plurality of interwoven threads form a plurality of open rectangular spaces for allowing enzyme ink to pass therethrough. The density and the size of the open spaces influence the amount of enzyme ink which becomes deposited on the conductive layer. Characteristics of the interwoven threads which influence the deposition of the enzyme ink are thread spacing and thread diameter. The thread spacing may range from about 90 threads per centimeter to about 120 threads per centimeter. The thread diameter may range from about 30 microns to about 50 microns. More specifically, in an embodiment, a screen suitable for screen printing an enzyme ink having ruthenium hexamine and glucose oxidase may have a thread spacing of about 120 threads per centimeter and a thread diameter of about 34 microns.

For test strip 100, adhesive layer 60 includes first adhesive pad 24, second adhesive pad 26, and third adhesive pad 28 as illustrated in Figure 1. Adhesive layer 60 may include a water based acrylic copolymer pressure sensitive adhesive which is commercially available from Tape Specialties LTD which is located in Tring, Herts, United Kingdom (part#A6435). Adhesive layer 60 is disposed on a portion of insulation layer 16, conductive layer 50, and substrate 5. Adhesive layer 60 binds hydrophilic layer 70 to test strip 100.

Hydrophilic layer 70 includes a distal hydrophilic portion 32 and proximal hydrophilic portion 34. Hydrophilic layer 70 may be a polyester having one hydrophilic surface such as an anti-fog coating which is commercially available from 3M.

For test strip 100, top layer 80 includes a clear portion 36 and opaque portion 38 as illustrated in Figure 1. Top layer 80 is disposed on and adhered to hydrophilic layer 70. Top layer 80 may be a polyester. It should be noted that the clear portion 36 substantially overlaps distal hydrophilic portion 32 which allows a user to visually confirm that the sample receiving chamber 92 may be sufficiently filled. Opaque portion 38 helps the user observe a high degree of contrast between a colored fluid such as, for example, blood within the sample receiving chamber 92 and the opaque portion 38 of top layer 80.

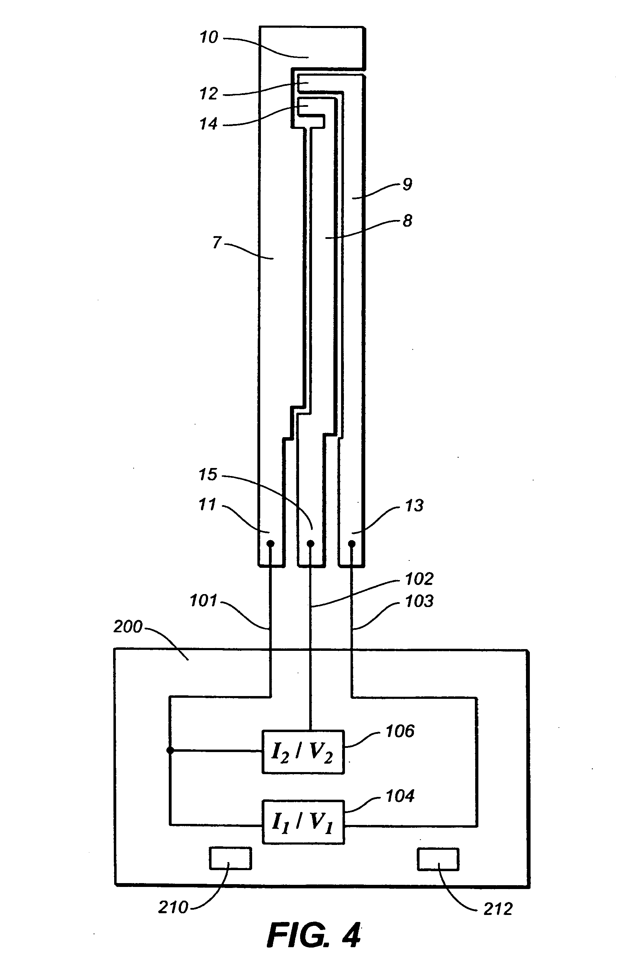

Figure 3 illustrates a test meter 200 suitable for connecting to test strip 100. Test meter 200 includes a display 202, a housing 204, a plurality of user interface buttons 206, and a strip port connector 208. Test meter 200 further includes electronic circuitry within housing 204 such as a memory 210, a microprocessor 212, electronic components for applying a test voltage, and also for measuring a plurality of test current values (see 104 and 106 in Figure 4). Proximal portion 4 of test strip 100 may be inserted into strip port connector 208. Display 202 may output a glucose concentration and also may be used to show a user interface for prompting a user on how to perform a test. The plurality of user interface buttons 206 allow a user to operate test meter 200 by navigating through the user interface software.

Figure 4 shows a simplified schematic of test meter 200 interfacing with test strip 100. Test meter 200 includes a first connector 103, second connector 102, and a reference connector 101 which respectively form an electrical connection to first contact 13, second contact 15, and reference contact 11. The three aforementioned connectors are part of strip port connector 208. When performing a test, a first test voltage source 104 applies a first test voltage *V₁* between first working electrode 12 and reference electrode 10. As a result of first test voltage *V₁*, test meter 200 may then measure a first test current *I₁*. In a similar manner, second test voltage source 106 applies a second test voltage *V₂* between second working electrode 14 and reference electrode 10. As a result of second test voltage *V₂*, test meter 200 may then measure a second test current *I₂*. In an embodiment, first test voltage *V₁* and second test voltage *V₂* may be about equal allowing a glucose measurement to be performed twice where a first measurement is performed with first working electrode 12 and a second measurement is performed with second working electrode 14. The use of two glucose measurements can increase accuracy by averaging the two results together. For simplifying the description of the following sections, the algorithms for determining an accurate glucose concentration will be described for only one working electrode and reference electrode. It should be apparent to one skilled in the art, that the invention should not be limited to one working electrode and reference electrode, but that multiple working electrodes can also be applied to the embodiments.

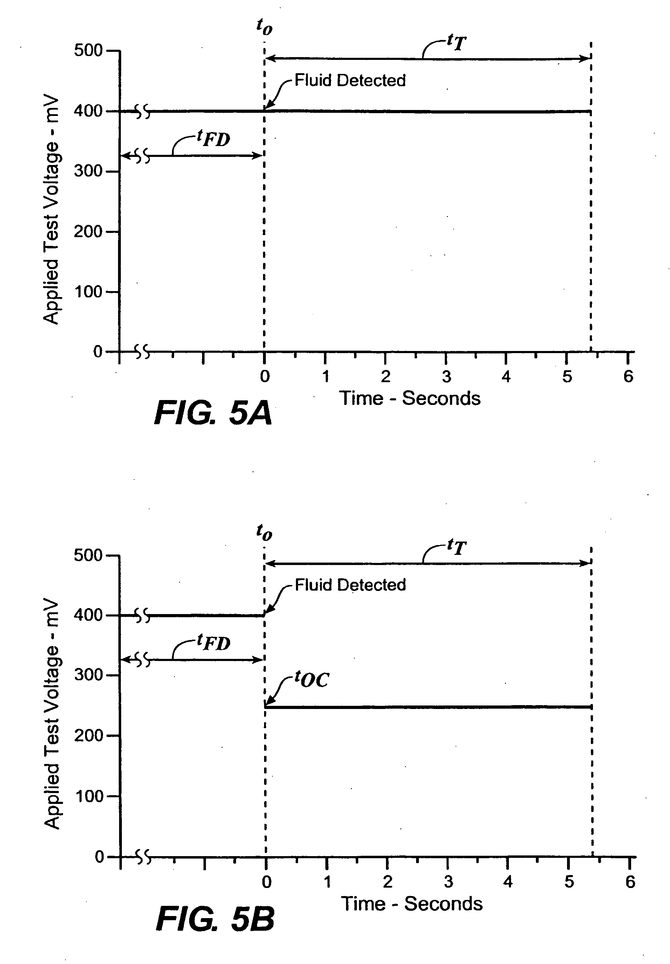

Figure 5a is a chart showing a test voltage that would be applied by test meter 200 to test strip 100 for a test time interval *t_{T}* which starts when physiological fluid is detected by test strip 100. In Figure 5a, the test voltage shown is 400 mV. As illustrated in Figure 5a, before the physiological fluid is applied, test meter 200 is in a fluid detection mode in which a fluid detection voltage may be 400 mV. It will be apparent to one skilled in the art that the test voltage and the fluid detection voltage can be different. In Figure 5a, the test meter is in a fluid detection mode during fluid detection time interval *t_{FD}* prior to the detection of physiological fluid at time *t₀*. In the fluid detection mode, test meter 200 determines when a fluid is applied to inlet 90 and pulled into sample receiving chamber 92 such that the fluid wets both first working electrode 12 and reference electrode 10. Note that first working electrode 12 and reference electrode 10 are effectively short-circuited when the physiological fluid contiguously covers both first working electrode 12 and reference electrode 10. Once test meter 200 recognizes that the physiological fluid has been applied because of, for example, a sufficient increase in the measured test current, test meter 200 assigns a zero second marker at time *t₀* and starts the test time interval *t_{T}*. For example, as shown in Figure 5a, test time interval *t_{T}* is about 5.4 seconds. Upon the completion of the test time interval *t_{T}*, the test voltage is removed.

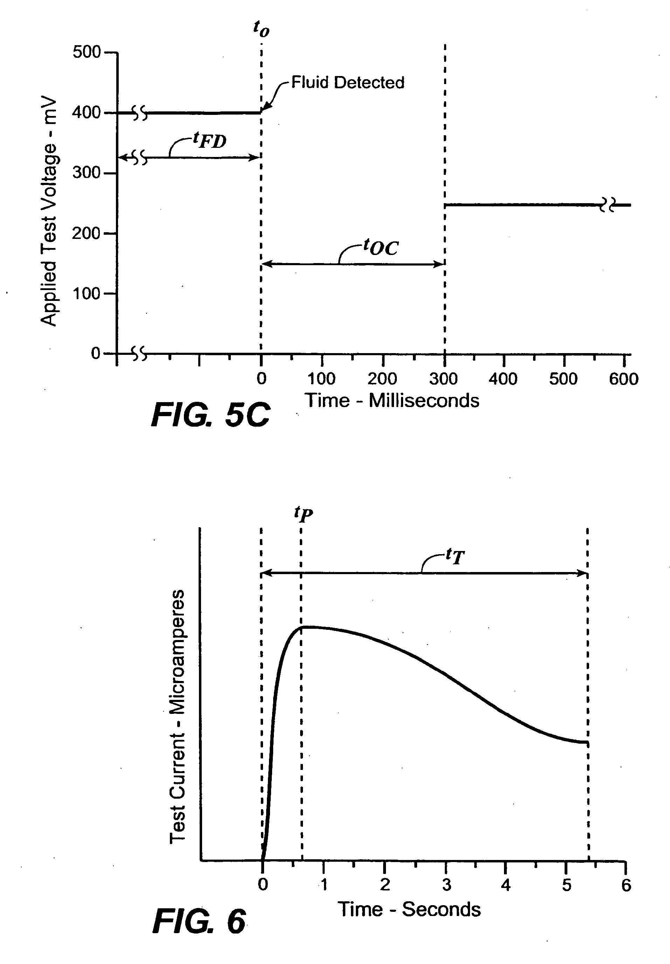

In an alternative embodiment, a test voltage waveform can be applied that includes an open-circuit time interval after fluid is detected in sample receiving chamber 92. For example, the test voltage as shown in Figure 5b is 400 mV during a fluid detection time interval *t_{FD}*. Once test meter 200 recognizes that the physiological fluid has been applied because, for example, the test current has increased to a level greater than a pre-determined threshold (e.g., about 50 nanoamperes) for a pre-determined time interval (e.g., about 20 milliseconds), test meter 200 assigns a zero second marker at time *t₀* and applies an open-circuit for an open-circuit time interval *t_{oc}*. For example, as shown in Figure 5c, the open-circuit time interval *t_{oc}* is about 300 milliseconds. Upon completion of the open-circuit time interval *t_{oc}*, the test meter applies a test voltage of about 250 millivolts for a time interval ranging from about 200 milliseconds to about 5.4 seconds.

In general, it is desirable to use a test voltage which is more positive than a redox voltage of the mediator used in the test strip. In particular, the test voltage should exceed the redox voltage by an amount sufficient to ensure that the resulting test current will not be dependent on small variations in the test voltage. Note that a redox voltage describes a mediator's intrinsic affinity to accept or donate electrons when sufficiently close to an electrode having a nominal voltage. When a test voltage is sufficiently positive with respect to the mediator's redox voltage, the mediator will be rapidly oxidized. In fact, the mediator will be oxidized so quickly at a sufficiently positive test voltage (i.e., limiting test voltage) that the test current magnitude will be limited by the diffusion of the mediator to the electrode surface (i.e., limiting test current). Where first working electrode 12 is a carbon ink and the mediator is ferricyanide, a test voltage of about +400 mV may be sufficient to act as a limiting test voltage. For an embodiment where first working electrode 12 is a carbon ink and the mediator is Ru^{III}(NH₃)₆, a test voltage of about +250 mV may be sufficient to act as a limiting test voltage. It will be apparent to one skilled in the art that other mediator and electrode material combinations will require different limiting test voltages.

A test meter that is designed to apply a limiting test voltage can have some variation in the applied test voltage without affecting the magnitude of the limiting test current. It is desirable for a test meter to apply a limiting test voltage because the test meter can be constructed with relatively inexpensive electronic components because it is not necessary to tightly control the test voltage. In summary, a test meter which applies a limiting test voltage can robustly measure a glucose concentration in an accurate and precise manner using low cost components.

Figure 6 is a chart showing the test current generated by test strip 100 during test time interval *t_{T}.* In general, the test current increases rapidly when test strip 100 is initially wetted with the physiological fluid and then forms a peak at a maximum peak time *tₚ*. After the peak maximum, the test current gradually decreases. The overall magnitude of the test currents will increase with increasing glucose concentration.

To produce sufficiently accurate and precise glucose concentration, test strip 100 should have a sufficiently reproducible and well defined electroactive area for first working electrode 12 and second working electrode 14. The magnitude of the test current is directly proportional to the area of one of the working electrodes. For instance, if the variation in the area of first working electrode 12 was high from one test strip to another, then the variation observed in the measured glucose concentrations will also be high when testing multiple test strips. Thus, it is important that the variation for first working electrode 12 and second working electrode 14 be relatively small so that the variation in the measured glucose concentration can, in turn, also be relatively small.

The area of first working electrode 12 and second working electrode 14, as shown in Figures 1 and 2, may be defined by the process of screen printing. A batch of test strips made by the process of screen printing can output sufficiently precise glucose concentration measurements (e.g., less than about a 5% CV). However, under certain circumstances and conditions, there may be a need to manufacture test strip batches which have even more precise electroactive areas. For instance, there has been a customer driven desire to design test strips which can measure glucose using very small volumes of blood (e.g., < 1 microliter). One of the theories being that the use of less blood will result in less pain during the glucose measuring procedure.

One strategy for designing test strips which have smaller sample volumes is to decrease the area of the first and second working electrode. However, as first and second working electrode and second working electrode become smaller, non-idealities in the screen printing process may start to significantly affect the precision of the working electrodes. For instance, variable stretching of a screen by a squeegee may affect the size of a working electrode. Additionally, a screen typically has a plurality of rectangular openings for deposing either a conductive layer or an insulation layer. The plurality of rectangular opening may cause an edge of either the conductive layer or insulation layer to be jagged especially when the size of the working electrode is comparable to the size of the rectangular opening. In the prior art embodiment as illustrated in Figures 1 and 2, first working electrode 12 has two sides 20 as defined by the screen printed conductive layer 50 and the other two sides 19 as defined by the screen printed insulation layer 16. It should be noted that insulation layer 16 is used to help cover the first working electrode track 9. Thus, there is a need to develop a process for manufacturing test strips which can have a more precise electrode areas that do not suffer from the disadvantages of screen printing. Further, there is a need to develop simpler manufacturing process which do not need an insulation layer for defining the electrode area and/or for covering the working electrode track.

In an embodiment, a test strip may be manufactured using a process of laser ablation for improving the accuracy and precision of the electroactive area of the first and second working electrode. The process of laser ablation on a conductive layer allows the edge definition of the electrode area to be better controlled than with other processes such as screen printing. In addition, the process of laser ablation may be used to substantially define the electrode area without the need of an insulation layer.

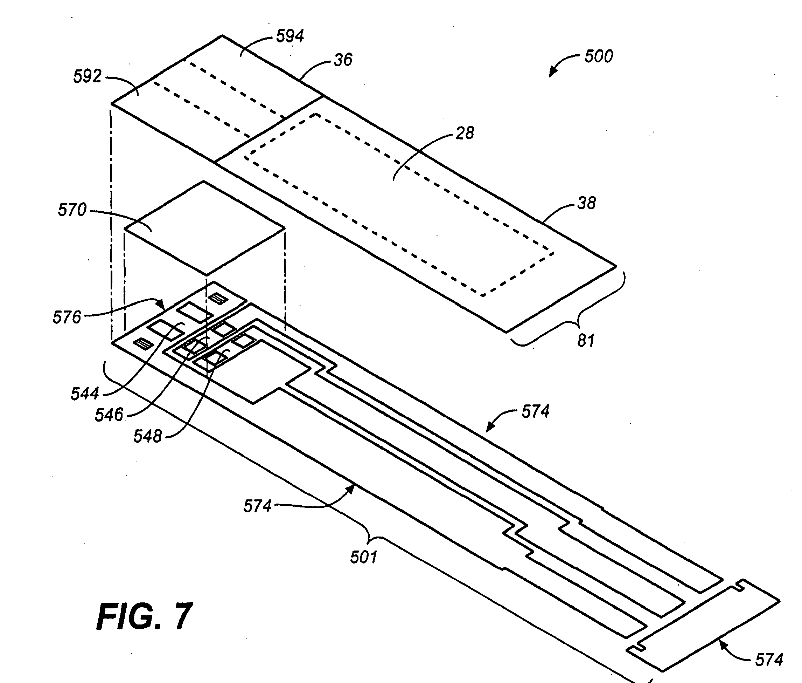

Figure 7 illustrates a top exploded perspective view of an unassembled test strip 500, which is an embodiment that can also utilize the previously described reagent formulation. Similar to test strip 100, test strip 500 includes a conductive layer 501, a reagent layer 570, and a top tape 81. Test strip 500 has a distal portion 576, a proximal portion 578, and two sides 574.

Although various embodiments described herein are particularly adapted to the measurement of a glucose concentration in blood, it will be apparent to those skilled in the art that the test strip described herein may be adapted to enable an improved precision for the electrochemical measurement of other analytes. Examples of other analytes that may be measured with the test strip embodiments are lactate, ethanol, cholesterol, amino acids, choline, hemoglobin, and fructosamine in blood.

### EXAMPLE 1

The reagent layer was formulated as an enzyme ink suitable for screen printing as follows. 100 ml of 200 mM aqueous phosphate buffer was adjusted to pH 7. A mixture was formed by adding 5 g of hydroxyethyl cellulose (HEC), 1 g of poly(vinyl pyrrolidone vinyl acetate) (PVP-VA S-630), 0.5 ml of DC 1500 Dow Corning antifoam to 100 mL of phosphate buffer and mixed by homogenization. The mixture was allowed to stand overnight to allow air bubbles to disperse and then used as a stock solution for the formulation of the enzyme ink. Next, 7.5 grams of Cab-o-Sil TS610 was gradually added by hand to the mixture until about 4/5 of the total amount of Cab-o-Sil TS610 had been added. The remainder Cab-o-Sil TS610 was added with mixing by homogenization. The mixture was then rolled for 12 hours. About 18 g of ruthenium hexamine ([Ru^{III}(NH₃)₆]Cl₃) was then added and mixed by homogenization until dissolved. Finally, 2.8 g of glucose oxidase enzyme preparation (250 Units/mg) was added and then thoroughly mixed into the solution. The resulting formulation was ready for printing, or could be stored with refrigeration.

### EXAMPLE 2

A first batch of test strips 100 was prepared using a ruthenium based reagent formulation as described in Example 1. A second batch of test strips 100 was prepared with a reagent formulation using a ferricyanide mediator instead of ruthenium hexamine in a manner similar to Example 1. A portion of the first and second batch of test strips 100 were stored at room temperature in a desiccated environment for 7 days. Another portion of the first and second batch of test strips 100 were stored at 40 °C in a 70% relative humidity environment for 7 days.

The first and second batch of test strips were tested in a test meter using a test voltage at +400 mV. Blood samples were tested which had a glucose concentration ranging from about 70 mg/dL to about 600 mg/dL.

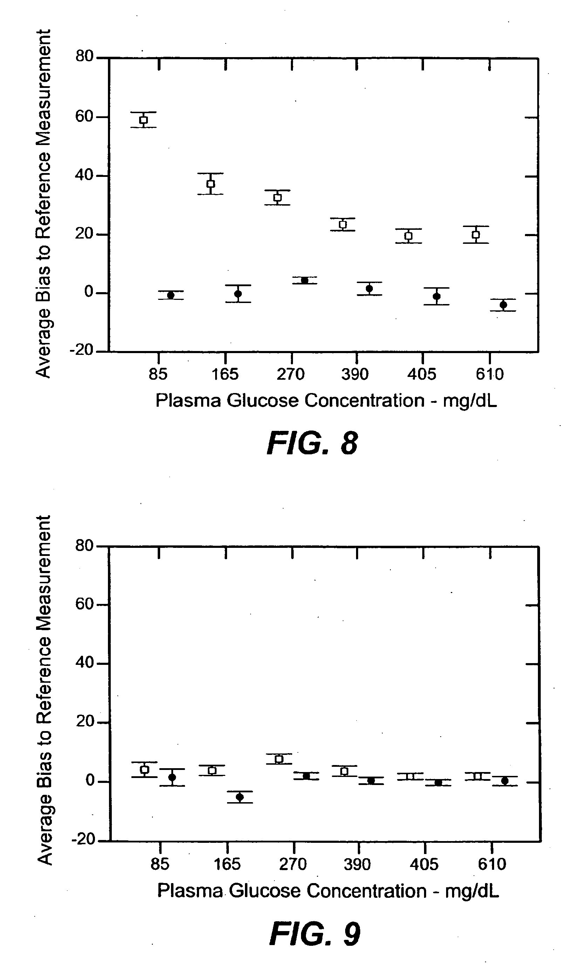

Figure 8 is a graph showing that average bias to reference measurement for the second batch of test strips had a positive bias to reference measurement when they were stored for seven days at 40 °C in a 75% relative humidity (RH) (open squares). In general, the positive bias to reference measurement was the largest at low glucose concentration which in this case was about a 60% bias to reference measurement at about 70 mg/dL glucose concentration. In contrast, the second batch of test strips which was stored at room temperature for seven days in a desiccated environment showed essentially no increase in bias to reference measurement, as illustrated by the filled in circles on Figure 8. Thus, test strips having ferricyanide which are exposed to relatively high humidity show an increase in bias to reference measurement.

Figure 9 is a graph showing that average bias to reference measurement for the first batch of test strips that were stored for seven days at 40 °C in a 75% relative humidity (RH) (open squares) or at room temperature in a desiccated environment (filled in circles) showed no overall increase in bias to reference measurement. Thus, test strips having ruthenium which are exposed to relatively high humidity show no increase in bias to reference measurement which is in contrast to test strip having ferricyanide.

### EXAMPLE 3

The first and second batch of test strips (as described in Example 2) were tested in a test meter using a test voltage at +400 mV. Blood samples were tested which had a glucose concentration of about 70 mg/dL and a uric acid concentration ranging from about 0 mg/dL to about 20 mg/dL.

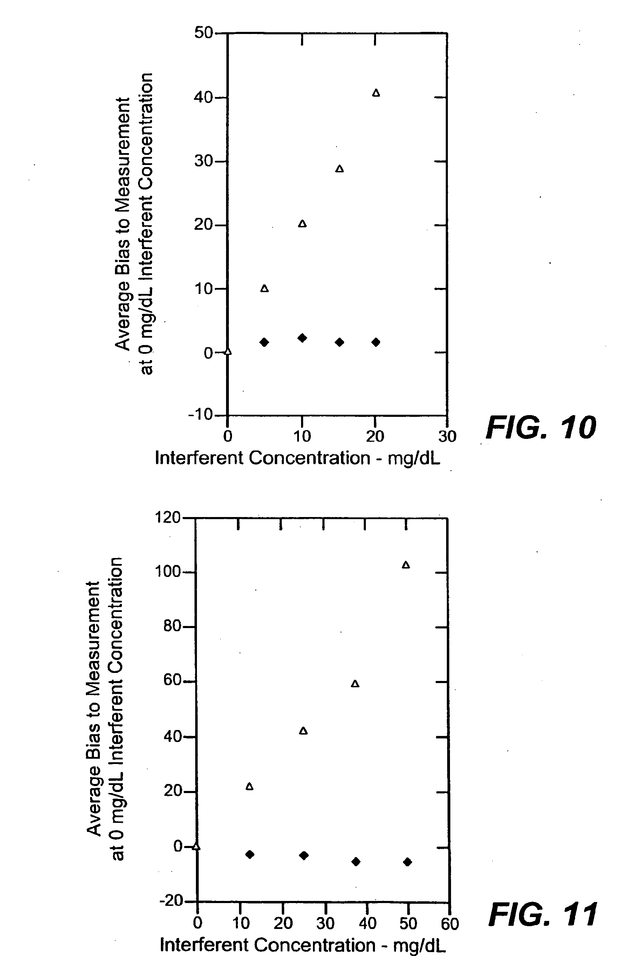

For the second batch of test strips as illustrated by open triangles in Figure 10, the average bias to reference measurement of the test strip measurements increased with increasing amounts of uric acid in an approximately linear manner. Therefore, test strips using a ferricyanide based reagent layer showed that uric acid, which is an interfering compound, can be oxidized by ferricyanide creating a non-glucose related increase in the test current.

For the first batch of test strips as illustrated by filled in diamonds in Figure 10, the average bias to reference measurement of the test strip measurements did not increase with increasing amounts of uric acid. Therefore, test strips using a ruthenium based reagent layer showed that uric acid, which is a potentially interfering compound, was not oxidized by ruthenium enabling a more glucose selective measurement.

### EXAMPLE 4

The first and second batch of test strips (as described in Example 2) were tested in a test meter using a test voltage at +400 mV. Blood samples were tested which had a glucose concentration of about 70 mg/dL and a gentisic acid concentration ranging from about 0 mg/dL to about 50 mg/dL.

For the second batch of test strips as illustrated by open triangles in Figure 11, the average bias to reference measurement of the test strip measurements increased with increasing amounts of gentisic acid in an approximately linear manner. Therefore, test strips using a ferricyanide based reagent layer showed that gentisic acid, which is an interfering compound, can be oxidized by ferricyanide creating a non-glucose related increase in the test current.

For the first batch of test strips as illustrated by filled in diamonds in Figure 11, the average bias to reference measurement of the test strip measurements did not increase with increasing amounts of gentisic acid. Therefore, test strips using a ruthenium based reagent layer showed that gentisic acid, which is a potentially interfering compound, was not oxidized by ruthenium enabling a more glucose selective measurement.

While the invention has been described in terms of particular variations and illustrative figures, those of ordinary skill in the art will recognize that the invention is not limited to the variations or figures described. In addition, where methods and steps described above indicate certain events occurring in certain order, it is intended that certain steps do not have to be performed in the order described but in any order as long as the steps allow the embodiments to function for their intended purposes.

## Claims

1. A formulation for coating a test strip, the formulation comprising:
an enzyme selected from the group consisting of glucose oxidase and glucose dehydrogenase;
a ruthenium hexamine mediator; and
a solution that dissolves the enzyme and the ruthenium hexamine mediator and in which the ruthenium hexamine comprises a concentration range from 15% to 20% of weight.

2. A test strip comprising:
a planar substrate that extends from a first end to a second end;
first and second electrodes disposed on the substrate proximate one of the first and second ends; and
a reagent layer having an enzyme selected from the group consisting of glucose oxidase and glucose dehydrogenase and ruthenium hexamine trichloride in a buffer solution with the ruthenium hexamine trichloride comprising a concentration range from 15% to 20% (weight / volume).

3. The formulation of Claim 1 or the test strip of Claim 2, wherein the enzyme is glucose oxidase and the glucose oxidase has an activity range from 1500 units/mL to 8000 units/mL.

4. The formulation of Claim 1, wherein the solution is a buffer selected from the group consisting of phosphate, citrate, and citraconate.

5. The test strip of Claim 2, wherein the buffer is selected from the group consisting of phosphate, citrate, and citraconate.

6. The formulation of Claim 4 or the test strip of Claim 5, wherein the buffer is phosphate and has a pH of 7.

7. The test strip of Claim 2, wherein the test strip comprises a working electrode and a reference electrode, and the reagent layer is disposed on the working electrode

8. The formulation of Claim 1, wherein the formulation further comprises a filler having hydrophilic and hydrophobic domains.

9. The test strip of Claim 2, wherein the reagent layer further comprises a filler having hydrophilic and hydrophobic domains.

10. The formulation of Claim 8 or test strip of Claim 9, wherein the filler comprises silica.

11. The test strip of Claim 9, wherein the reagent layer is disposed on the working electrode using a process of screen printing, wherein the screen has a plurality of interwoven threads, the plurality of interwoven threads forming a plurality of open rectangular spaces for allowing the formulation to pass therethrough, the plurality of interwoven threads having a thread spacing and a thread diameter, wherein the thread spacing ranges from 90 threads per centimeter to 120 threads per centimeter and the thread diameter may range from 30 microns to 50 microns.

## Patentansprüche

1. Formulierung zur Beschichtung eines Teststreifens, wobei die Formulierung umfasst:
ein Enzym, das ausgewählt ist aus der Gruppe, bestehend aus Glucose-Oxidase und Glucose-Dehydrogenase;
einen Rutheniumhexamin-Vermittler; und
eine Lösung, die das Enzym und den Rutheniumhexamin-Vermittler löst und in der das Rutheniumhexamin einen Konzentrationsbereich von 15 bis 20 Gew.-% umfasst.

2. Teststreifen, umfassend:
ein planares Substrat, das sich von einem ersten Ende zu einem zweiten Ende erstreckt;
erste und zweite Elektroden, die auf dem Substrat nahe zu einem von den ersten und zweiten Enden angeordnet sind; und
eine Reagensschicht mit einem Enzym, das ausgewählt ist aus der Gruppe, bestehend aus Glucose-Oxidase und Glucose-Dehydrogenase und Rutheniumhexamintrichlorid in einer Pufferlösung, wobei das Rutheniumhexamintrichlorid einen Konzentrationsbereich von 15% bis 20% (Gewicht/Volumen) umfasst.

3. Formulierung nach Anspruch 1 oder Teststreifen nach Anspruch 2, wobei das Enzym Glucose-Oxidase ist und die Glucose-Oxidase einen Aktivitätsbereich von 1500 Einheiten/ml bis 8000 Einheiten/ml besitzt.

4. Formulierung nach Anspruch 1, wobei die Lösung ein Puffer ist, der ausgewählt ist aus der Gruppe, bestehend aus Phosphat, Citrat und Citraconat.

5. Teststreifen nach Anspruch 2, wobei der Puffer ausgewählt ist aus der Gruppe, bestehend aus Phosphat, Citrat und Citraconat.

6. Formulierung nach Anspruch 4 oder Teststreifen nach Anspruch 5, wobei der Puffer Phosphat ist und einen pH von 7 besitzt.

7. Teststreifen nach Anspruch 2, wobei der Teststreifen eine Arbeitselektrode und eine Referenzelektrode umfasst und die Reagensschicht auf der Arbeitselektrode abgeschieden ist.

8. Formulierung nach Anspruch 1, wobei die Formulierung weiter einen Füllstoff mit hydrophilen und hydrophoben Domänen umfasst.

9. Teststreifen nach Anspruch 2, wobei die Reagensschicht weiter einen Füllstoff mit hydrophilen und hydrophoben Domänen umfasst.

10. Formulierung nach Anspruch 8 oder Teststreifen nach Anspruch 9, wobei der Füllstoff Siliciumdioxid umfasst.

11. Teststreifen nach Anspruch 9, wobei die Reagensschicht auf der Arbeitselektrode unter Verwendung eines Siebdruckverfahrens abgeschieden ist, wobei das Sieb eine Mehrzahl von ineinander verwobenen Fäden besitzt, wobei die Mehrzahl der ineinander verwobenen Fäden eine Mehrzahl von offenen rechtwinkligen Räumen bildet, um zu ermöglichen, dass die Formulierung dort hindurchgeht, die Mehrzahl von ineinander verwobenen Fäden einen Fadenzwischenraum und einen Fadendurchmesser aufweist, wobei der Fadenzwischenraum von 90 Fäden pro Zentimeter bis 120 Fäden pro Zentimeter reicht und der Fadendurchmesser von 30 Mikrons bis 50 Mikrons reichen kann.

## Revendications

1. Formule devant être appliquée sur une bandelette réactive, la formule comprenant :
un enzyme sélectionné dans le groupe constitué par du glucose oxydase et du glucose déshydrogénase ;
un médiateur d'hexamine ruthénium ; et
une solution qui dissout l'enzyme et le médiateur ruthénium hexamine, et dans laquelle l'hexamine ruthénium comprend une concentration comprise dans une plage de 15 % à 20 % en poids.

2. Bandelette réactive comprenant :
un substrat planaire qui s'étend d'une première extrémité à une seconde extrémité ;
des première et seconde électrodes qui sont placées sur le substrat à proximité de l'une des première et seconde extrémités ; et
une couche réactive comprenant un enzyme sélectionné dans le groupe constitué par du glucose oxydase et du glucose déshydrogénase et du trichlorure d'hexamine ruthénium dans une solution tampon, le trichlorure d'hexamine ruthénium comprenant une concentration comprise dans une plage de 15 % à 20 % (poids/volume).

3. Formule selon la revendication 1 ou bandelette réactive selon la revendication 2, dans laquelle l'enzyme est du glucose oxydase et le glucose oxydase a une plage d'activité allant de 1500 unités/ml à 8000 unités/ml.

4. Formule selon la revendication 1, dans laquelle la solution est une solution tampon sélectionnée dans le groupe constitué par du phosphate, du citrate et du citraconate.

5. Bandelette réactive selon la revendication 2, dans laquelle la solution tampon est sélectionnée dans le groupe constitué par du phosphate, du citrate et du citraconate.

6. Formule selon la revendication 4 ou bandelette réactive selon la revendication 5, dans laquelle la solution tampon est du phosphate et elle a un pH de 7.

7. Bandelette réactive selon la revendication 2, dans laquelle la bandelette réactive comprend une électrode de travail et une électrode de référence, et la couche réactive est placée sur l'électrode de travail.

8. Formule selon la revendication 1, dans laquelle la formule comprend par ailleurs une charge ayant des domaines hydrophile et hydrophobe.

9. Bandelette réactive selon la revendication 2, dans laquelle la couche réactive comprend par ailleurs une charge ayant des domaines hydrophile et hydrophobe.

10. Formule selon la revendication 8 ou bandelette réactive selon la revendication 9, dans laquelle la charge comprend de la silice.

11. Bandelette réactive selon la revendication 9, dans laquelle la couche réactive est placée sur l'électrode de travail au moyen d'un procédé d'impression à l'écran de soie, dans lequel l'écran comprend une pluralité de fils tissés entre eux, la pluralité de fils tissés entre eux formant une pluralité d'espaces rectangulaires ouverts qui permettent à la formule de passer au travers, la pluralité de fils tissés entre eux ayant un écartement entre fils et un diamètre de fil, dans laquelle les écartements entre fils se situent dans une plage allant de 90 fils par centimètre à 120 fils par centimètre, et le diamètre de fil peut se situer dans une plage allant de 30 microns à 50 microns.
